# EUROPEAN PATENT APPLICATION

(11) **EP 3 238 668 A1**
(43) Date of publication of application: **01.11.2017**
(21) Application number: 15872697.6
(22) Date of filing: 07.12.2015
(51) Int. Cl.: A61F 2/915, A61F 2/88

(54) **STENT**

(30) Priority: 26.12.2014 JP 2014266471
(71) Applicant: Terumo Kabushiki Kaisha, Tokyo 151-0072 (JP)
(72) Inventor: SHINTAKU, Yutaro, Tokyo 163-1450 (JP); KUMAZAWA, Takashi, Ashigarakami-gun Kanagawa 259-0151 (JP); SEKINE, Yuusuke, Ashigarakami-gun Kanagawa 259-0151 (JP)
(74) Representative: Casalonga
(86) International application number: PCT/JP2015/084319
(87) International publication number: WO 2016/104128

(57) **Abstract**

[Task] To provide a stent which can prevent the overall stent from randomly collapsing when expanded, even if the stent is formed of a biodegradable polymer.

[Means for Resolution] A stent 100 has a configuration which is formed into a cylindrical shape by a linear strut 101. The strut has outer peripheral portions 102 extending around an axial direction of the cylindrical shape and a connection portion 103 connecting the outer peripheral portions to each other in a gap formed by the outer peripheral portions, and has a configuration in which the outer peripheral portions and the connection portion are integrally formed of a biodegradable polymer. In addition, a portion of the strut includes a fragile portion 104 which is more fragile than other different portions of the strut, and in a state where the fragile portion includes a component the same as that of the other portions, the biodegradable polymer itself is fragile.

## Description

### [Technical Field]

The present invention relates to a stent.

### [Background Art]

A stent is a medical device used for securing a cavity by widening a stenosed or occluded site in order to treat various diseases caused by a stenosed or occluded lumen of a blood vessel. The stent has a substantially cylindrical shape whose outer periphery is formed in a mesh shape, and expands outward in a radial direction so that an expansion holding force for securing the cavity is applied to the lumen.

The stent is used for not only a linear portion of the lumen, but also a bifurcated portion of the lumen. When the stent is used for the bifurcated portion, for example, as disclosed in PTL 1, a portion of the stent is broken, and an outer peripheral gap portion of the stent is broadened, thereby securing a route for communicating with the lumen of a bifurcated target.

### [Citation List]

### [Patent Literature]

[PTL 1] JP-A-2011-245001

### [Summary of Invention]

### [Problem that the Invention is to Solve]

However, in a case where the stent formed of a biodegradable polymer is used for the bifurcated portion, if an excessive force is forcibly applied when the outer peripheral gap portion of the stent is broadened, since the biodegradable polymer is generally weaker in strength than metal, not only a portion of the stent is broken, but also the overall stent randomly collapses. As a result, there is a possibility that a desired expansion holding force may not be applied to the lumen.

The present invention is made in view of this problem, and an object thereof is to provide a stent which can prevent the overall stent from randomly collapsing when expanded, even if the stent is formed of a biodegradable polymer.

### [Means for Solving the Problem]

In order to achieve the above-described object, a stent according to the present invention has a configuration which is formed into a cylindrical shape by a linear strut. The strut has outer peripheral portions extending around an axial direction of the cylindrical shape and a connection portion connecting the outer peripheral portions to each other in a gap formed by the outer peripheral portions, and has a configuration in which the outer peripheral portions and the connection portion are integrally formed of a biodegradable polymer. In addition, a portion of the strut includes a fragile portion which is more fragile than other different portions of the strut, and in a state where the fragile portion includes a component the same as that of the other portions, the biodegradable polymer itself is fragile.

### [Advantageous Effects of Invention]

According to the stent configured as described above, since the fragile portion is broken, a strong force is less likely to be applied to other portions different from the fragile portion. Therefore, it is possible to prevent the overall stent from randomly collapsing when expanded, even if the stent is formed of the biodegradable polymer.

### [Brief Description of the Drawings]

[Fig. 1] Fig. 1 is a perspective view of a stent according to the embodiment.
[Fig. 2] Fig. 2 is a development view in which a portion of an outer periphery of the stent according to the embodiment is developed by being cut into a linear shape along an axial direction.
[Fig. 3] Fig. 3 is a partially enlarged view illustrating an enlarged fragile portion.
[Fig. 4] Fig. 4 is a stent development view illustrating an arrangement of the fragile portions in the overall stent, and a state where a portion having no fragile portion includes a helical shape.
[Fig. 5] Fig. 5 is a perspective view illustrating a state where the stent according to the embodiment is mounted on a balloon catheter.
[Fig. 6] Fig. 6 is a perspective view illustrating a state where the stent according to the embodiment is expanded by a balloon.
[Fig. 7] Fig. 7 is a perspective view illustrating a state where the balloon is deflated after the stent is expanded.
[Fig. 8] Fig. 8 is a perspective view illustrating a state where the balloon is inserted into an outer peripheral gap of the expanded stent according to the embodiment.
[Fig. 9] Fig. 9 is a partially enlarged view illustrating an enlarged outer periphery of the stent into which the balloon is inserted.

### [Best Mode for Carrying Out the Invention]

Hereinafter, an embodiment according to the present invention will be described with reference to the drawings. Dimensional proportions in the drawings are exaggerated and different from actual proportions for convenience of description.

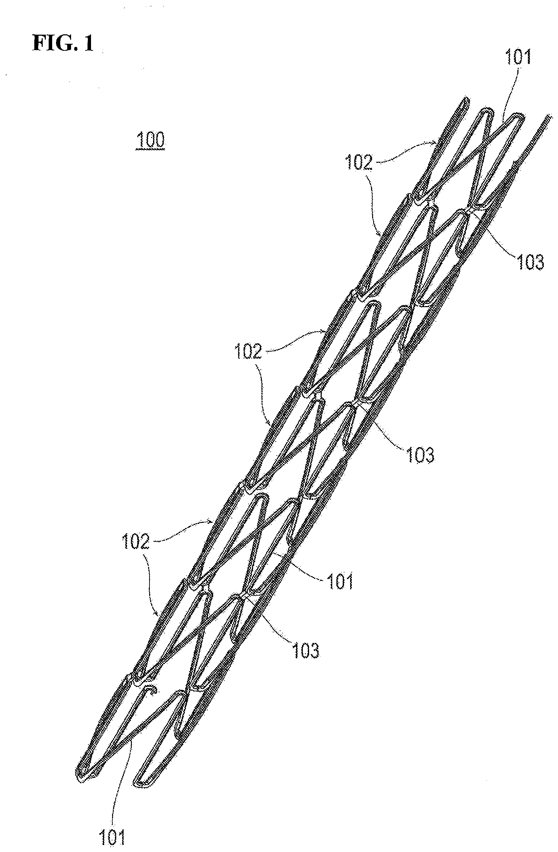

As illustrated in Fig. 1, a stent 100 according to the embodiment has a strut 101 which is a linear configuration element. The stent 100 has a configuration in which a cylindrical shape is formed by the strut 101. The strut 101 includes outer peripheral portions 102 extending around an axial direction of the cylindrical shape, and a connection portion 103 connecting the outer peripheral portions 102 to each other.

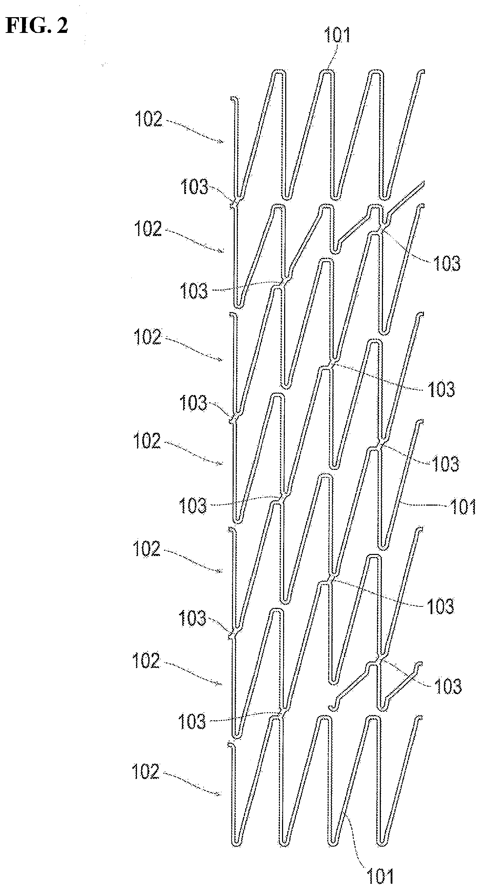

As illustrated in Fig. 2, the connection portion 103 is disposed in a gap formed between the outer peripheral portions 102.

For example, the outer peripheral portion 102 includes at least any one of a helical portion extending in a helical shape around the axial direction (vertical direction in Fig. 2) of the stent 100, and endless annular portions extending around the axial direction of the stent 100. For example, the annular portions are disposed in both ends in the axial direction of the stent 100, and the helical portion is disposed therebetween. However, an arrangement of the annular portion and the helical portion is not particularly limited.

The connection portion 103 connects the outer peripheral portions 102 to each other so as to be coaxially aligned in the axial direction of the stent 100. An arrangement of the connection portions 103 is not particularly limited. In addition, the number of the connection portions 103 is not particularly limited.

The outer peripheral portion 102 and the connection portion 103 are integrally formed of a biodegradable polymer. Here, the biodegradable polymer is a polymer which gradually biodegrades inside a living body. As long as the polymer does not adversely affect the inside of the living body of a human being or an animal, the biodegradable polymer is not particularly limited.

For example, it is preferable that the biodegradable polymer is at least one polymer selected from a group including aliphatic polyester, polyester, polyanhydrides, polyorthoester, polycarbonate, polyphosphazenes, polyphosphate ester, polyvinyl alcohol, polypeptides, polysaccharide, protein, and cellulose, a copolymer obtained by optionally copolymerizing a monomer configuring the above-described polymer, and a mixture of the polymers and/or the copolymers (here, the mixture means a broad concept including a compound such as a polymer alloy). Among these, it is preferable to use the aliphatic polyester since the aliphatic polyester is less reactive in a biological tissue and can control degradation inside the living body.

For example, without being particularly limited, the aliphatic polyester includes at least one polymer selected from a group including polylactic acid, polyglycolic acid, and polycaprolactone, a copolymer obtained by optionally copolymerizing the monomer configuring the above-described polymer, and the mixture of the polymers and/or the copolymers.

For example, a weight-average molecular weight of the overall strut 101 including the outer peripheral portion 102 and the connection portion 103 is 10,000 or greater, but a configuration is not limited thereto. The weight-average molecular weight of the overall strut 101 is preferably 10,000 to 1,000,000, more preferably 20,000 to 500,000, and particularly preferable 50,000 to 200,000.

For example, a method of measuring the weight-average molecular weight includes GPC, a light scattering method, a viscosity measurement method, and TOF mass spectrometry (TOFMASS).

The stent 100 has a configuration in which the overall strut 101 is integrally formed of the biodegradable polymer. Accordingly, after fulfilling a function as the stent 100, that is, after restraining a rate of blood vessel occlusion and restenosis in an acute phase, the stent 100 vanishes after being degraded and absorbed in the living body. Therefore, there is a low risk in restenosis and thrombotic complications in a last stage.

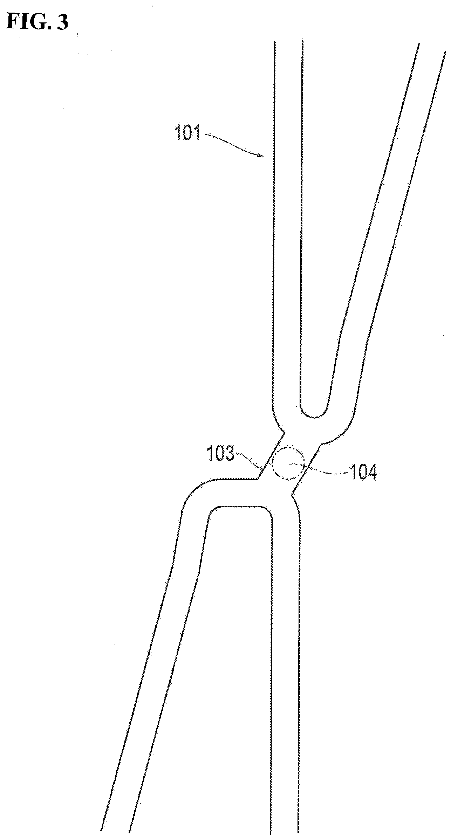

As illustrated in Fig. 3, a fragile portion 104 is formed in at least one connection portion 103. The fragile portion 104 is formed so as to be more fragile than other portions in the strut 101. In addition, the fragile portion 104 may be formed in a portion of the connection portion 103, and the overall connection portion 103 may be formed using the fragile portion 104. Any configuration may be adopted as long as the connection portion having the fragile portion is more likely to be broken than the other connection portion having no fragile portion.

For example, the fragile portion 104 is formed by causing the biodegradable polymer itself to be fragile without forming a portion of the strut 101 by adding a material having a component different from that of the biodegradable polymer forming the strut 101 as a separate member, or without forming a notch or hole shape in a portion of the strut 101. As long as the fragile portion 104 is formed so that the biodegradable polymer itself is fragile while including the component the same as that of other portions of the strut 101, a configuration is not particularly limited.

For example, the fragile portion 104 is formed so that the weight-average molecular weight of a portion of the strut 101 is smaller than the weight-average molecular weight of the overall strut 101. In this case, the weight-average molecular weight of the fragile portion 104 is preferably 5% to 50% of the weight-average molecular weight of the overall strut 101.

For example, the weight-average molecular weight of the fragile portion 104 may be reduced in such a way that energy of an electron beam, a radiation beam, an infrared beam, or heat is locally converged to the strut 101.

In addition, the fragile portion 104 may be formed by causing the biodegradable polymer itself to be fragile in such a way that a compounding ratio of the component of the biodegradable polymer in the fragile portion 104 is differently changed from a compounding ratio of the component in other portions of the strut 101.

As an example in this case, in the strut 101 formed of a copolymer of polylactic and polyglycolic acid, it is conceivable to increase the compounding ratio of polyglycolic acid in the fragile portion 104 compared to other portions.

The weight-average molecular weight of the fragile portion 104 or the compounding ratio of the component is differently changed from other portions as described above. In this manner, a biodegradation rate of the fragile portion 104 in addition to strength can be changed. For example, if the weight-average molecular weight of the fragile portion 104 is reduced, the fragile portion 104 vanishes after being degraded earlier than other portions.

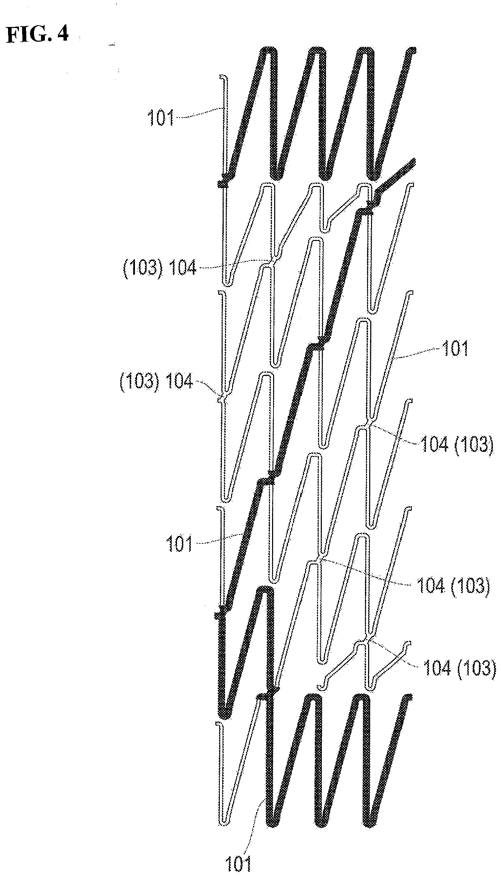

As illustrated in Fig. 4, according to the present embodiment, the fragile portion 104 is formed in a plurality of the connection portions 103. The strut 101 includes a helical shape (thick black line in Fig. 4) which ceaselessly continues from one end to the other end in the axial direction (vertical direction in Fig. 4) while avoiding the fragile portion 104. That is, the fragile portion 104 is not disposed in a portion having the helical shape. The fragile portion 104 is not formed in the portion having the helical shape illustrated by the thick black line in Fig. 4. Accordingly, the portion is less likely to be broken.

Next, a method form of using the stent 100 will be described.

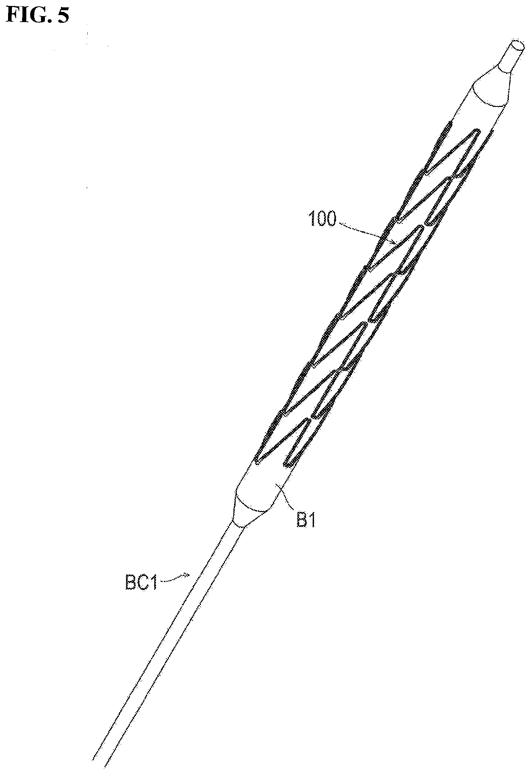

As illustrated in Fig. 5, in a state where the stent 100 is mounted on a balloon B1 of a balloon catheter BC1, the stent 100 is delivered to a bifurcated portion of a lumen such as a blood vessel. In this case, the balloon B1 is deflated, and the stent 100 is contracted. A known one in the related art can be used as the balloon catheter BC1. In addition, the balloon catheter BC1 and the stent 100 can be delivered to a desired position inside the lumen by using a known manual skill in the related art. Accordingly, detailed description thereof will be omitted.

The lumen for using the stent 100 is not limited to the blood vessel, and may be a biliary duct, a bronchial tube, an esophagus, other gastrointestinal tracts, and a urethra, for example.

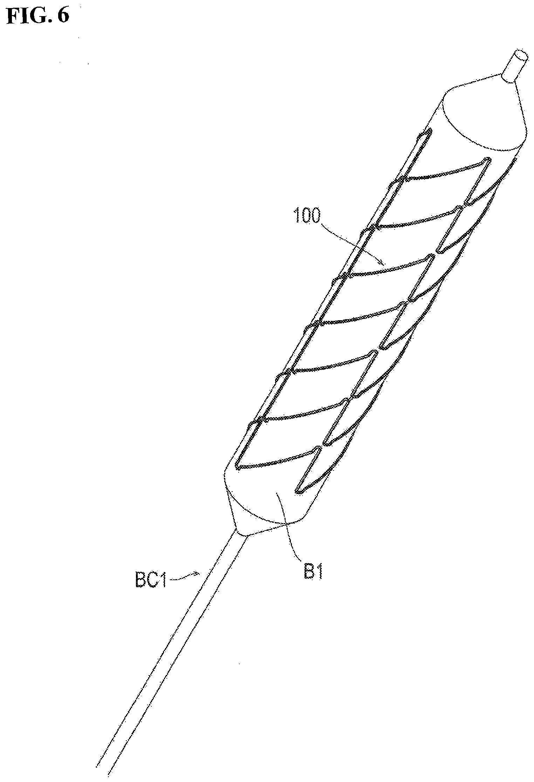

After the stent 100 is delivered to the bifurcated portion of the lumen, the balloon B1 is dilated as illustrated in Fig. 6. The balloon B1 expands the stent 100, and widens a stenosed site or an occluded site appearing in the bifurcated portion.

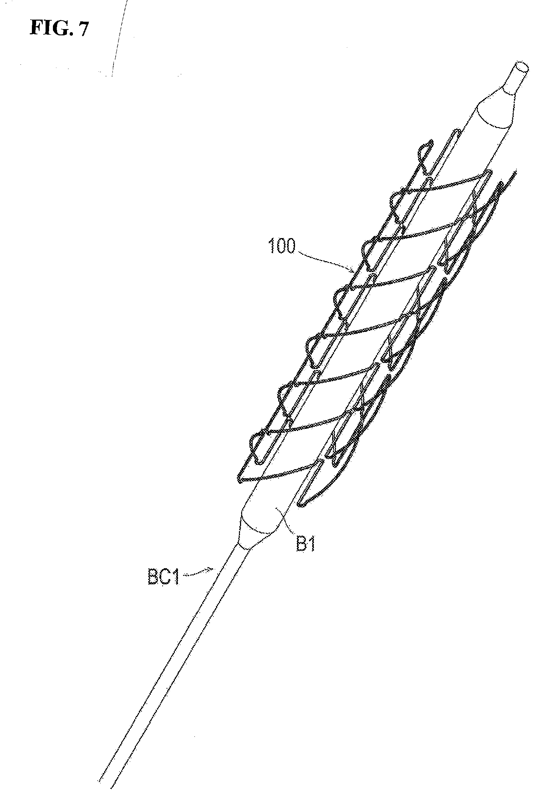

As illustrated in Fig. 7, after the stent 100 is expanded, the balloon B1 is deflated. In this case, the stent 100 maintains an expanded state. The expanded stent 100 secures a cavity by applying an expansion holding force to the lumen.

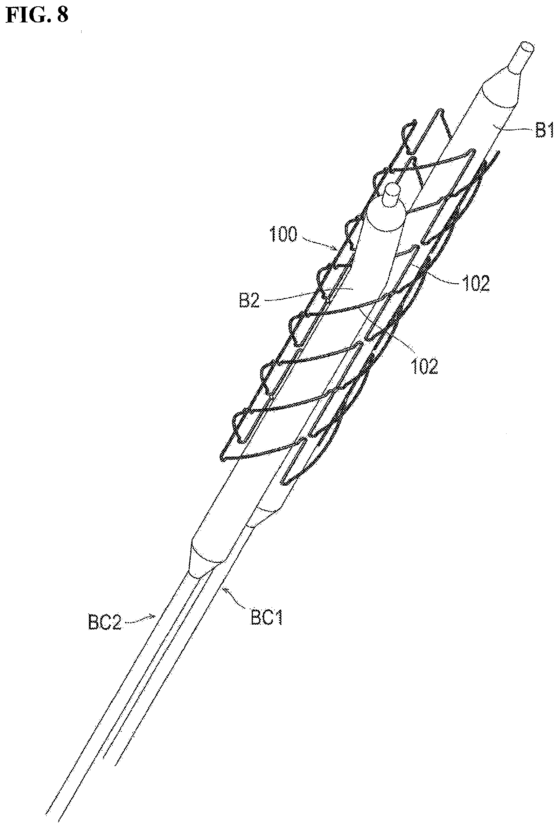

Thereafter, as illustrated in Fig. 8, a balloon B2 of another balloon catheter BC2 is inserted into a gap formed by the outer peripheral portion 102 of the stent 100. The balloon B2 is oriented in a direction of the other lumen bifurcated from the lumen into which the balloon B1 is inserted. As the balloon catheter BC2, it is possible to use a known one in the related art.

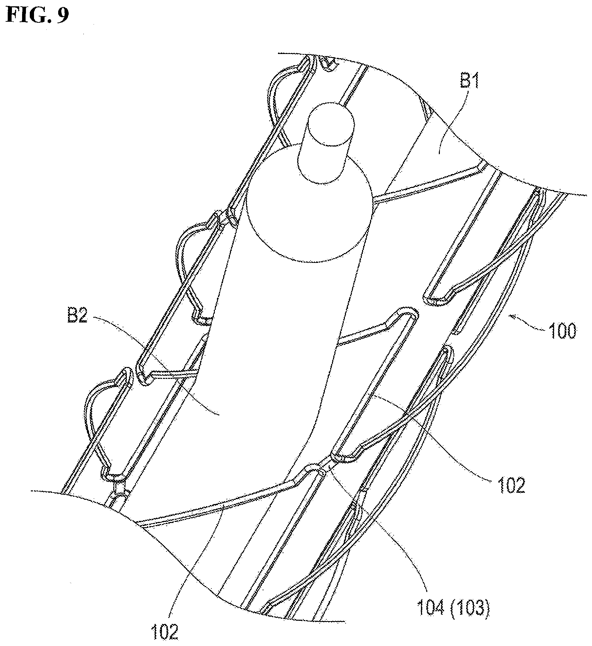

As illustrated in Fig. 9, the balloon B2 dilates and applies a tensile force to the outer peripheral portion 102 and the fragile portion 104 which are located around the balloon B2. When this force is applied thereto, the fragile portion 104 is broken. As a result, the gap formed by the outer peripheral portion 102 is broadened, thereby forming a large opening portion. In this manner, a medical device such as the catheter is likely to be delivered to the lumen serving as a bifurcated target to which the balloon B2 is oriented.

The balloon B2 is dilated, and the balloon B1 is also dilated, thereby holding the stent 100 by pressing the stent 100 against an inner wall of the lumen.

Next, an operation effect according to the present embodiment will be described.

In the stent 100, since the fragile portion 104 is broken, a strong force is less likely to be applied to other portions different from the fragile portion 104, for example, such as the outer peripheral portion 102 and the connection portion 103 having no fragile portion 104. Therefore, it is possible to prevent the overall stent 100 from randomly collapsing when expanded, even if the stent 100 is integrally formed of the biodegradable polymer.

The fragile portion 104 is formed in the connection portion 103 instead of the outer peripheral portion 102. Accordingly, the fragile portion 104 is restrained from vanishing due to the broken or degraded outer peripheral portion 102. Therefore, the stent 100 can effectively apply the expansion holding force to the lumen from the outer peripheral portion 102.

As illustrated by the thick black line in Fig. 4, the strut 101 includes a helical shape which continues from one end to another end in the axial direction (vertical direction in Fig. 4) while avoiding the fragile portion 104. Therefore, even if the fragile portions 104 vanish by being broken or preferentially degraded at a plurality of locations, the strut 101 maintains a state where the strut 101 is connected from one end to another end in the axial direction. Therefore, the stent 100 is prevented from being broken, thereby enabling the stent 100 to stably fulfill its own function.

When the weight-average molecular weight of the fragile portion 104 is equal to or smaller than 50% of the weight-average molecular weight of the overall strut 101, the strength of the fragile portion 104 is further reduced compared to other portions of the strut 101. Accordingly, the fragile portion 104 can be easily broken.

When the weight-average molecular weight of the overall strut 101 is 10,000 or greater, the strength is ensured for the overall stent 100. Accordingly, the stent 100 can stably hold the widened lumen.

The compounding ratio of the component of the biodegradable polymer is set so that the fragile portion 104 and other portions of the strut 101 have mutually different compounding ratios. In this manner, when the strength of the fragile portion 104 is weakened, the fragile portion 104 can be easily broken.

Without being limited to the above-described embodiment, the present invention can be modified in various ways within the scope of the appended claims.

For example, without being limited to the connection portion, the location for forming the fragile portion may be formed in a portion of the strut which is different from the connection portion, for example, such as the outer peripheral portion.

In addition, the location for using the stent according to the present invention is not limited to the bifurcated portion of the lumen. The stent according to the present invention may be used for a location having no bifurcation in the lumen, for example, such as a linear site.

This application is based upon and claims the benefit of priority of Japanese Patent Application No. 2014-266471 filed on December 26, 2014, the contents of which are incorporated herein by reference in its entirety.

### [Reference Signs List]

100 STENT,
101 STRUT,
102 OUTER PERIPHERAL PORTION,
103 CONNECTION PORTION,
104 FRAGILE PORTION,
BC1, BC2 BALLOON CATHETER,
B1, B2 BALLOON

## Claims

1. A stent having a configuration which is formed into a cylindrical shape by a linear strut,
wherein the strut has outer peripheral portions extending around an axial direction of the cylindrical shape and a connection portion connecting the outer peripheral portions to each other in a gap formed by the outer peripheral portions, and has a configuration in which the outer peripheral portions and the connection portion are integrally formed of a biodegradable polymer, and
wherein a portion of the strut includes a fragile portion which is more fragile than other different portions of the strut, and in a state where the fragile portion includes a component the same as that of the other portions, the biodegradable polymer itself is fragile.

2. The stent according to Claim 1,
wherein the fragile portion is formed in the connection portion.

3. The stent according to Claim 1 or 2,
wherein the strut includes a helical shape which ceaselessly continues from one end to the other end in the axial direction of the cylindrical shape while avoiding the fragile portion.

4. The stent according to any one of Claims 1 to 3,
wherein a weight-average molecular weight of the fragile portion is equal to or smaller than 50% of a weight-average molecular weight of the overall strut.

5. The stent according to any one of Claims 1 to 4,
wherein the weight-average molecular weight of the overall strut is 10,000 or greater.

6. The stent according to any one of Claims 1 to 5,
wherein a compounding ratio of the component of the biodegradable polymer in the fragile portion is different from a compounding ratio of the component in the other portions of the strut.
